# EUROPEAN PATENT SPECIFICATION

(11) **EP 0 493 347 B1**
(45) Date of publication and mention of the grant of the patent: **07.05.1997**
(21) Application number: 91830575.6
(22) Date of filing: 19.12.1991
(51) Int. Cl.: C07C 7/12, B01J 20/02, C07C 9/04

(54) **Process for the purification of methane**
Verfahren zur Reinigung von Methan
Procédé pour la purification de méthane

(30) Priority: 20.12.1990 IT 2245590
(43) Date of publication of application: 01.07.1992
(73) Proprietor: SAES GETTERS S.p.A., I-20123 Milano (IT)
(72) Inventor: Succi, Marco, Milano (IT); Solcia, Carolina, Grezzano (Milano) (IT)
(74) Representative: Adorno, Silvano

(56) References cited:
- EP-A- 0 365 490
- EP-A- 0 470 936
- DE-B- 2 461 759
- GB-A- 1 478 083
- US-A- 4 312 669
- PATENT ABSTRACTS OF JAPAN vol. 11, no. 394 (C-465)(2841) 23 December 1987
- VUOTO,1990, Vol XX, No 2, pages 299-302

## Description

The present invention relates to a method or process for the removal of impurities from CH₄ and particularly for the removal of N₂ and O₂.

The gas methane (CH₄) is widely used in industry as, for example, in the production of thin films of artificial diamonds.

The purity of the gas is of great importance. While it is not possible to say what impurity level is associated with a given degree or number of defects found in the layers or films, it is possible to say that the higher the purity of gas used the greater is the yield of such films.

In the past it has been common practice to use organic hydrocarbons or plastic materials for the removal of impurities from hydrogen-containing gases such as CH₄. However their use has led to one or more disadvantages.

With organic hydrocarbons or plastic sorbents an increase of the operating temperature can cause the release of unwanted hydrocarbons with subsequent recontamination of the purified gas. The very nature of the materials is such that they may decompose and lead to the production of unwanted gases.

GB-A-1478083 and JP-A-62-153389 describe the use of plastic semipermeable membranes for the separation of CH₄, respectively, from a mixture CH₄-H₂S - CO₂ and from a mixture CH₄ - H₂ - N₂ - CO - CO₂; organic membranes however show the same draswbacks as organic sorbents. German Patent N° 2.461.759 discloses a method for the separation of CH₄ from a mixture of gases by selective adiabatic adsorption of impurities such as H₂O, CO₂ and hydrocarbons over a bed of an adsorbing porous material such as silica gel .

The use of hydrided getter metal or alloy such as those disclosed in EP-A-470 936 has prevented the sorption of some of the undesirable gases. Furthermore the use of hydrided getters implies the introduction of additional costs and possible difficulties.

It is therefore an object of the present invention to provide an improved process for the removal of impurity gases, in particular of N₂ and O₂, from impure CH₄.

It is another object of the present invention to provide an improved process for the removal of impurities from impure CH₄ which does not require the use of organic supports or plastic materials.

It is a further object of the present invention to provide an improved process for the removal of impurity gases from impure CH₄ which does not require the use of a hydrided getter metal or alloy.

Yet another object of the present invention is to provide an improved process for the removal of gaseous impurities from impure CH₄ which does not require temperatures which would dissociate the methane.

According to the invention these objects are achieved through a process having the features of claim 1. In particular for removing impurity gases from nitrogen-free impure CH₄ the process steps will show the features of claim 6.

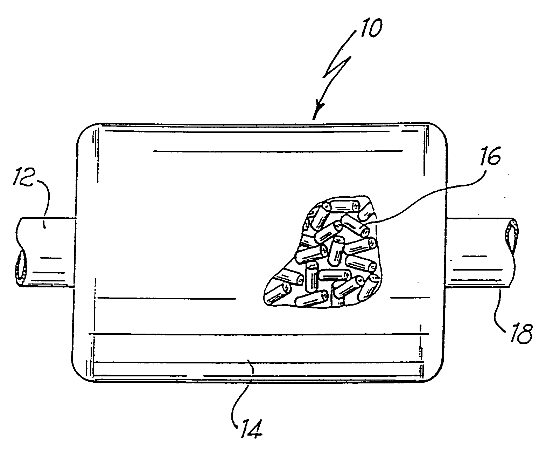

These and other advantages and objects of the present invention will become clear to those skilled in the art by reference to the following description and the single drawing which is a schematic diagram, partially cut away, of an apparatus useful in a process of the present invention.

The invention provides a process for the removal of impurity gases from impure CH₄. It comprises the steps of heating an alloy of Zr-V-Fe to a temperature of from 300°C to 950°C.

The alloy is then cooled to a temperature of less than or equal to 400°C and greater than 100°C, below which there is no appreciable sorption of the CH₄. At this temperature the impurity containing CH₄ is brought into contact with the Zr-V-Fe alloy.

If N₂ is not one of the impurities the alloy may be Zr₂Fe.

With reference to the drawing, there is illustrated an apparatus 10 useful in carrying out the process of the present invention for the removal of impurity gases from impure CH₄. Apparatus 10 comprises an impure gas inlet 12 through which enters the impure CH₄. Inlet 12 is in fluid communication with a chamber 14 for the purification of the CH₄ and chamber 14 contains a getter alloy 16. Chamber 14 is in fluid communication with a purified gas outlet 18. Getter alloy 16 is in the form of a powder of particle size preferably less than 500 µm and most preferably less than 125 µm. It is preferably in the form of pellets, with or without a binder, suitable for incorporation within chamber 14.

The composition of the alloy is Zr-V-Fe.

Typical compositions of the ternary alloys according to the invention comprise :
- Zr: from 45 to 75, and preferably from 47 to 70% by weight
- V: from 20 to 50, and preferably from 24 to 45% by weight
- Fe: from 5 to 35, and preferably from 5 to 10% by weight.

The compositions in weight percent when plotted on a ternary composition diagram in weight percent Zr, weight percent V and weight percent Fe lie within a polygon having as its corners the points defined by:
(a) 75% Zr - 20% V - 5% Fe
(b) 45% Zr - 20% V - 35% Fe
(c) 45% Zr - 50% V - 5% Fe
and preferably lie within the polygon having as its corners the points defined by:
(d) 70% Zr - 25% V - 5% Fe
(e) 70% Zr - 24% V - 6% Fe
(f) 66% Zr - 24% V - 10% Fe
(g) 47% Zr - 43% V - 10% Fe
(h) 47% Zr - 45% V - 8% Fe
(i) 50% Zr - 45% V - 5% Fe.

Alloys useful in the present invention are described in US-A-4,312,669.

It should be realized that small amounts of other metals can be used without substantially altering its purification characteristics. For instance the iron may be partially replaced by nickel, or the vanadium may be partially replaced with niobium. It may be advantageous to replace some of the zirconium with titanium without substantially altering the main sorption ability of the basic ternary alloy. One or more substitutions may take place at the same time.

The temperature of activation of the Zr-V-Fe alloy should be high enough to clean the surface of the particles from oxides and nitrides that have formed during air exposure. Activation can be accomplished by heating the getter alloy to an elevated temperature for a time sufficient to thermally break up the sorption-inhibiting coating that forms on the alloy during handling in air. This sorption-inhibiting coating typically comprises oxides from atmospheric oxygen and nitrides from atmospheric nitrogen. The elevated temperature can be from about 300 to about 950°C. At the lower end of the range the heating should occur for longer than 10 minutes. Twenty minutes is generally sufficient. Longer heating times waste energy but do not adversely affect performance. At the upper end of the range the alloy should be heated for at least 30 seconds. Two minutes is generally sufficient. Longer heating times waste energy but do not adversely affect performance. As a guide heating at 400°C for four hours is sufficient. Similarly 500°C for about 40 minutes is sufficient. The heating should, of course, be accomplished in the absence of oxygen, nitrogen and other active gases. This can be accomplished by heating in a vacuum or in a rare gas such as argon or at the lower temperatures, in methane.

The alloy, when sorbing impurities from CH₄ must not cause its dissociation and must be held at a temperature below the temperature of appreciable dissociation of CH₄. This temperature should be less than or equal to 400°C and preferably 350°C. It should be greater than 100°C.

If N₂ is not an impurity then the alloy can be Zr₂Fe.

The invention may be better understood by reference to the following examples wherein all parts and percentages are by weight unless otherwise indicated. These examples are designed to teach those skilled in the art how to practice the present invention and represent the best mode presently known for practicing the invention.

### EXAMPLE 1

A chamber 14 of an apparatus 10 was filled with 70g of pellets, 4 mm in diameter and 3 mm in height, of compressed Zr-V-Fe powder. The nominal powder composition was 70% Zr - 24.6% V - 5.4% Fe by weight.

The getter alloy was heated to 350°C for 4 hours, in a flow of CH₄, for activation.

Then a flow of CH₄ containing 7.6 ppm (parts per million) of oxygen and 7.0 ppm of nitrogen was passed through the chamber 14 at a flow rate of 100 cm³/minute.

The level of oxygen at the outlet was measured using an oxygen analyzer MKIII/Y made by OSAKA OXYGEN having a sensitivity of two parts per billion (ppb) of O₂. The level of nitrogen at the outlet was measured using a gas chromatograph with a meta-stable helium detector having a sensitivity of 20 ppb.

No oxygen was detected at the outlet while the nitrogen level had decreased by 90%. After 7 days of continuous flow no significant variations were observed.

### EXAMPLE 2

Example 1 was repeated exactly with the sole exception that the pellets were replaced by compressed powder pellets of Zr₂Fe.

When the impure methane was caused to flow measurements at the outlet revealed no traces of oxygen. After 7 days of continuous flow again no significant variations were observed. The level of nitrogen however remained constant at 7 ppm.

Although the invention has been described in considerable detail with reference to certain preferred embodiments designed to teach those skilled in the art how best to practice the invention, it will be realized that other modifications may be employed without departing from the spirit and scope of the appended claims.

## Claims

1. A process for the removal of N₂ and O₂ from impure CH₄, characterized by comprising the steps of:
A. activating a getter alloy chosen from the group consisting of Zr-V-Fe and Zr₂Fe alloys at a temperature of between 300°C and 950°, for a time comprised respectively between more than 10 minutes and at least 30 seconds;
B. bringing the alloy to a temperature which is less than or equal to 400°C and greater than 100°C; and
C. contacting the impure CH₄ with the Zr-V-Fe or Zr₂Fe alloy.

2. A process according to claim 1 which comprises passing impure CH₄ through a chamber (14) having an impure gas inlet (12) and a purified gas outlet (18), said chamber (14) containing an alloy of Zr-V-Fe, characterized by comprising the steps of:
A. activating the Zr-V-Fe alloy in vacuum or in a flow of rare or inert gas or CH₄ at a temperature between 400°C and 950°C, for a time comprised between more than 10 minutes and at least 30 seconds, respectively, to make it gas sorptive;
B. cooling the alloy to a temperature ≤400°C and >100°C; and
C. contacting the impure CH₄ with the Zr-V-Fe alloy.

3. A process according to claim 2 in which step A takes place at 400°C for 4 hours.

4. A process according to claim 2 in which step B takes place at 350°C.

5. A process according to claim 1 for the removal of O₂ from nitrogen-free impure CH₄, characterized by comprising the steps of:
A. activating an alloy of Zr₂Fe by heating it to a temperature of between 300°C and 950°C for a time comprised between more than 10 minutes and at least 30 seconds, respectively;
B. bringing the alloy to a temperature ≤400°C and >100°C; and
C. contacting nitrogen-free, impure CH₄ with the Zr₂Fe alloy.

## Patentansprüche

1. Verfahren zum Entfernen von N₂ und O₂ aus verunreinigtem CH₄, **gekennzeichnet** durch die folgenden Schritte:
A. Aktivierung einer Getterlegierung, ausgewählt aus der Gruppe bestehend aus Zr-V-Fe und Zr₂Fe-Legierungen bei Temperaturen zwischen 300 °C und 950 °C für eine Zeit zwischen mehr als 10 Minuten und wenigstens 30 Sekunden;
B. Bringen der Legierung auf eine Temperatur von weniger oder gleich 400 °C und größer als 100 °C; und
C. Kontaktieren des verunreinigten CH₄ mit der Zr-V-Fe oder Zr₂Fe-Legierung.

2. Verfahren nach Anspruch 1, daß das Passieren von verunreinigtem CH₄ durch eine Kammer (14) umfaßt, welche einen Einlaß (12) für verunreinigtes Gas und ein Auslaß (18) für gereinigtes Gas besitzt und die eine Legierung aus Zr-V-Fe enthält, **gekennzeichnet** durch die Schritte:
A. Aktivieren der Zr-V-Fe-Legierung in Vakuum oder in einem Fluß von Edel- oder Inertgas oder CH₄ bei einer Temperatur zwischen 400 und 950 °C für eine Zeit zwischen mehr als 10 Minuten und wenigstens 30 Stunden, um es gasadsorptiv zu machen;
B. Kühlen der Legierung auf eine Temperatur von ≤ 400 °C und > 100 °C; und
C. Kontaktieren des verunreinigten CH₄ mit der Zr-V-Fe-Legierung.

3. Verfahren nach Anspruch 2, bei dem der Schritt A bei 400 °C für 4 Stunden stattfindet.

4. Verfahren nach Anspruch 2, bei dem der Schritt B bei 350 °C stattfindet.

5. Verfahren nach Anspruch 1 zur Entfernung von O₂ aus sticksstofffreiem verunreinigtem CH₄, **gekennzeichnet** durch die Schritte:
A. Aktivieren einer Legierung aus Zr₂Fe durch Erhitzen auf eine Temperatur zwischen 300 und 950 °C für eine Zeit zwischen mehr als 10 Minuten und wenigstens 30 Sekunden;
B. Bringen der Legierung auf eine Temperatur ≤ 400 °C und > 100 °C; und
C. Kontaktieren des stickstofffreien verunreinigten CH₄ mit der Zr₂Fe-Legierung.

## Revendications

1. Un procédé pour le retrait de N₂ et de O₂ contenus dans du CH₄ impur, caractérisé en ce qu'il comprend les opérations consistant à :
A. activer un alliage absorbant choisi dans le groupe comprenant des alliages de Zr-V-Fe et de Zr₂Fe à une température entre 300°C et 950°, pendant une durée comprise respectivement entre plus de 10 minutes et au moins 30 secondes ;
B. amener l'alliage à une température qui est inférieure ou égale a 400°C et supérieure a 100°C ; et
C. mettre en contact le CH₄ impur avec l'alliage de Zr-V-Fe ou de Zr₂Fe.

2. Un procédé selon la revendication 1 qui comprend le fait de faire passer le CH₄ impur à travers une chambre (14) présentant une entrée de gaz impur (12) et une sortie de gaz purifié (18), ladite chambre (14) contenant un alliage de Zr-V-Fe, caractérisé en ce qu'il comprend les opérations consistant à :
A. activer l'alliage de Zr-V-Fe sous vide ou dans un écoulement de gaz rare ou inerte ou de CH₄ à une température entre 400°C et 950°C, pendant une durée comprise respectivement entre plus de 10 minutes et au moins 30 secondes, pour rendre le gaz sorbant ;
B. refroidir l'alliage à une température ≤ 400°C et > 100°C ; et
C. mettre en contact le CH₄ impur avec l'alliage de Zr-V-Fe.

3. Un procédé selon la revendication 2, dans lequel l'opération A prend place à 400°C pendant 4 heures.

4. Un procédé selon la revendication 2, dans lequel l'opération B prend place à 350°C.

5. Un procédé selon la revendication 1 pour le retrait du O₂ contenu dans du CH₄ impur sans azote, caractérisé en ce qu'il comprend les opérations consistant à :
A. activer un alliage de Zr₂Fe en le chauffant à une température entre 300°C et 950°C pendant une durée comprise respectivement entre plus de 10 minutes et au moins 30 secondes ;
B. amener l'alliage à une température ≤ 400°C et > 100°C ; et
C. mettre en contact, avec l'alliage de Zr₂Fe, le CH₄ impur sans azote.
